# EUROPEAN PATENT SPECIFICATION

(11) **EP 2 845 612 B1**
(45) Date of publication and mention of the grant of the patent: **13.05.2020**
(21) Application number: 14183523.1
(22) Date of filing: 04.09.2014
(51) Int. Cl.: A61L 27/60, A61L 27/56, A61L 27/20, A61L 27/24, C25D 1/18

(54) **Process for manufacturing self-standing films made of natural or synthetic macromolecules characterized by controlled porosity micropattern.**
Prozess zur Herstellung stabilen Filmen enthaltend natürliche oder synthetische Makromoleküle, charakterisiert durch ihr kontrolliertes Porositäts-Mikromuster.
Procédé de fabrication de films auto-portés faits de polymères naturels ou synthétiques présentant un micro-motif poreux.

(30) Priority: 06.09.2013 IT MI20131468
(43) Date of publication of application: 11.03.2015
(73) Proprietor: NextMaterials S.r.l., 20124 Milan (IT); Politecnico di Milano, 20133 Milano (IT)
(72) Inventor: De Nardo, Luigi, 20154 Milan (IT); Altomare, Lina, 20133 Milan (IT); Cigada, Alberto, 20123 Milano (IT); Chiesa, Roberto, 22029 Uggiate Trevano (CO) (IT); Varoni, Elena Maria, 13900 Biella (IT); Rimondini, Lia, 40137 Bologna (IT)
(74) Representative: Gregorj S.r.l.

(56) References cited:
- EP-A1- 2 554 365
- WO-A2-2011/106526
- LI-QUN WU ET AL: "Biofabrication with biopolymers and enzymes: Potential for constructing scaffolds from soft matter", THE INTERNATIONAL JOURNAL OF ARTIFICIAL ORGANS, vol. 34, no. 2, 7 March 2011 (2011-03-07), pages 215-224, XP055114935, ISSN: 0391-3988, DOI: 10.5301/IJAO.2011.6406

## Description

### Technical field of the invention

The invention relates to a process for manufacturing two-dimensional self-standing structures (films), with a variable and controlled thickness, made of natural or synthetic, pH-dependent, polyelectrolyte macromolecules, characterized by controlled porosity micro-patterns.

More particularly, the invention relates to an electrochemical/electrophoretic process for the deposition in a controlled manner on a conductive micro-patterned template - preferably grids in conductive material such as graphite or metal, having suitable size/dimensions of the meshes lumen- of macromolecular polyelectrolytes, among which, as an illustrative, yet incomplete example, chitosan, alginate, collagen, in order to achieve self-standing films characterized by controlled porosity micro-patterns made of said macromolecules.

The invention also relates to the materials obtained via the above-mentioned process.

The process of the invention finds industrial application, as an illustrative, yet incomplete example, in the field of porous biomaterials, systems for drug-release and biosensors, as well as for the deposition of biopolymers and biological products.

### State of the art

From the technical and scientific literature and from the industrial experience, it is well known that the main therapeutic strategy for the treatment of skin lesions, and in many cases also of deep skin lesions, is the use of bandages. These are typically made of natural materials obtained via conventional techniques of tissue processing (spinning and subsequent weaving). Some devices are manufactured using non-woven fabrics.

In case of irreversible damage of the skin, the most common therapeutic approach is represented by autologous skin grafts (Atiyeh, Costagliola 2007; Metcalfe, Ferguson 2007). However, in case the lesions are very large (as in major burn cases) or with impaired regenerative capabilities (as in the case of diabetic ulcers), the donor sites may be insufficient. In these cases, treatments based on tissue regeneration approach are required.

For both therapeutic approaches, over the years, several devices have been designed and marketed, characterized by appropriate chemical-physical and morphologic characteristics. From the morphological point of view, these structures can be of two types: continuous, possibly characterized by a random porosity but controlled in size and interconnection at the micrometer scale, or micro-patterned. There are different techniques that enable to obtain morphologies belonging to either the first or the second category: the most suitable technology has to be selected according to the application.

Many technologies have been proposed in order to manufacture bandages and porous structures made of chitosan, based on: release of porogen agents, 3D printing, phase separation, film casting, electrospinning, emulsion, and lyophilization (But, Wang et al. 2001; Kumbar, James et al. 2008; Hutmacher 2001 Ho, Kuo et al., 2004; Madihally, Matthew 1999).

Solvent casting is one of the most commonly used techniques. Via this technique, a manufactured product is obtained from a solution of the polymer in a suitable solvent and subsequent evaporation in a mould. This technique can be modified to obtain porosity by adding a porogen agent, which is not soluble in the solvent of the polymer and which is soluble in non-solvents of the polymer itself. After the polymer solvent has evaporated, the extraction of the porogen is performed by immersion in a solution of non-solvents for the polymer. The size and shape of the pores depend on the porogen agent. In case the porogen agent is a gas, the technique is called gas-foaming.

As an illustrative example, porous structures have been made of chitosan by Ma et al. (2001) . This approach is interesting for its high versatility and the possibility of using the obtained materials in order to understand the microstructural effect in the response of biological tissues in contact therewith; however, this technique does not allow the production of large quantities of material.

Another commonly used technique for making scaffolds is lyophilisation. Madihally et al. (1999) have shown how structures of variable and controlled densities can be prepared from a solution of chitosan in acetic acid. Lyophilisation is a technique that allows the total elimination of water (or more generally of a solvent) from which samples are reduced in dehydrated powder (or porous solids). Lyophilisation is carried out at very low temperatures and under vacuum, so that the water (or the solvent) crystallizes and it sublimes, passing from the solid state to a vapour. However, these aspects make the actual industrial implementation very limiting, from the technological point of view, in case continuous surfaces of significant size are required.

The electrospinning is a technique that enables to obtain matrices having micrometric or nanometric fibres by means of electrostatic forces. A polymer solution is dripped by a syringe pump: on the surface of the drop, charges of the same sign are formed, which give rise to repulsive electrostatic forces. When these forces exceed the surface tension of the drop, a polymer jet is formed. Once formed, the jet undergoes the attraction of the electric field towards the collector. The jet between the spinneret and the collector passes through an area of instability wherein it is stretched in a decisive manner, leading to the formation of the nanofiber. Eventually, the nanofiber will deposit on the collection system (collector). This technique is a simple and inexpensive tool because it requires a reduced amount of material and it is applicable to a wide range of polymers. It allows controlling the diameter and the properties of the fibres by acting on the deposition parameters. Among its main disadvantages, there is the impossibility to manufacture structures showing a regular pattern, such as the spatial arrangement of through-holes and size/dimension thereof. A similar drawback is reported for more conventional techniques for the production of non-woven fabrics. Desai et al. (2008) obtained chitosan fibres by electrospinning.

Another technique widely used for the production of 3D structures with controlled morphology and porosity is 3D printing. This technology is based on dispensing of a binder, through a print nozzle, on a bed of polymer powder. Without the use of tooling, the object is sequentially built layer by layer: low viscosity liquid binder (solvent, solvent solution) is dispensed from a print nozzle on a thin bed of powders; drops, binding to the powder, compact and form a 2D layer. After a sheet has been printed and dried, the platform is lowered at a fixed distance, a new layer of powder is deposited, and the printing process is repeated. After the entire structure is printed, the unbound powders are eliminated. This technique can be performed at room temperature and allows incorporating biological agents, such as cells and growth factors, if the binder is not toxic (e.g. water). Hutmacher (2001) reports obtaining biodegradable scaffolds through 3D printing of powders of chitosan and starch mixtures and chitosan, starch and hydroxyapatite mixtures.

This technology, however, has major limitations on its applicability in industrial scale, somewhat impractical, making it not competitive.

LI-QUN WU et al. "Biofabrication with biopolymers and enzymes: Potential for constructing scaffolds from soft matter" THE INTERNATIONAL JOURNAL OF ARTIFICIAL ORGANS, vol. 34, no. 2, 7 march 2011, pages 215-224*,* discloses a study showing the possibility of electrochemical pattern transfer on amino-polysaccharide chitosan. Specifically, they micro-fabricated gold patterns on silicon wafers, casted a chitosan film onto the patterned wafers, immersed these complex structure on a aqueous phenol solution and transferred the pattern via an anodic polarization that electrochemically initiate the phenol reaction cascade.

However, even if very effective in terms of spatial resolution, the approach proposed by Wu et all. suffer of different limitations. It can be applied only to chitosan; the sequence of steps, involving the formation of a continuous film via solvent casting, limit the process time; the electrochemically initiated reactions lead to modification of the chitosan film's chemistry, structure and chitosan color, resulting in a dark brown; the electrochemically initiated modification of the chitosan film is localized to the interfacial region between the film and the anode.

EP 2 554 365 A1 is aimed at realizing electrodes for biomedical applications, a porous structure, in which the electrode is a patterned polymer material, composed by conventional conductive polymers (see claim 6) that by electro polymerization is deposited, i.e. coated onto or embedded into, the surface of a porous body while the porous body is composed of hydrogel that is not obtained via electrochemical/electrophoretic process.

EP 2 554 365 A1 discloses a process wherein a pattern that is composed of conductive polymer is electro polymerized and deposited, i.e. coated onto or embedded into, the surface of a porous body such as a soft gel (page 3, [0017 -0018]).

According to the foregoing, there was the strong need to achieve a process able to overcome all the limitations of the prior art in order to achieve easily, quickly, not requiring complex equipment and very short processing times, self-standing polymer films characterized by a controlled porosity micro-patterns. Therefore, such products can be used as components for the manufacturing of bandages, of supports for the regeneration and repair of biological tissues, for biosensors, for the transduction of signals, for the biofunctionalization of surfaces, and to create vesicles and composite coatings for tissue engineering also of complex surfaces.

### Summary of the invention

The applicants, surprisingly and unexpectedly, have achieved a process of preparation of self-standing films with variable and controlled thickness, characterized by controlled through-porosity micro-patterns, made of natural or synthetic pH-dependent polyelectrolyte macromolecules, the process comprising: a) cathodic and/or anodic electrochemical/electrophoretic deposition of said natural or synthetic macromolecules, in particular natural polysaccharides and/or proteins, more particularly chitosan and/or

alginate and/or collagen, considered individually or in combination, on a working electrode in the form of "micro-patterned conductive template", having suitable size/dimensions of the lumen of the meshes, preferably in a conductive material such as graphite or metal, in particular titanium or steel, preferably in the form of grid, the electrode immersed in an electrolyte solution, preferably an aqueous solution, comprising said macromolecules; b) mechanical removal of the self-standing films with variable and controlled thickness, characterized by a controlled through-porosity micro-patterns obtained in step a) from the working electrode.

The films obtained by the process according to claim 1 of the present invention are characterized by pores or through-porosity (through-channels), oriented perpendicular to the film plane and with a regular and even geometry in the same plane (Micro-pattern), said pores or through-porosity being controlled and controllable in shape, distribution in micrometer-sized channels, and spacing thereof. The material constituting the films obtained by the process according to claim 1 of the present invention being further characterized by a random, non-through porosity (secondary porosity), with lower pore size than the channels or through-pores, said secondary porosity resulting from the process of film deposition according to the present invention.

### Description of the drawings

Figure 1. Scanning electron microscopy of chitosan deposited on circular specimens of titanium.
Figure 2. Optical Microscopy of chitosan deposited on metal grids.
Figure 3. Scanning electron microscopy of chitosan deposited on metal grids.
Figure 4. Morphology of the chitosan coatings/deposits of chitosan obtained on grids with lumen size of 700 µm as a function of the deposition time (time) and the applied current density. Images are obtained by means of optical microscopy.
Figure 5. Morphology of chitosan coatings/deposits of chitosan obtained on grids with lumen size of 700 µm as a function of the deposition time (time) and the current density used. Images are obtained by means of optical microscopy.
Figure 6. Morphology of chitosan coatings/deposits of chitosan obtained on grids with lumen size equal to 560 µm as a function of the deposition time (time) and the current density used. Images are obtained by means of optical microscopy.
Figure 7. Morphology of chitosan coatings/deposits of chitosan obtained on grids with lumen size equal to 430 µm as a function of the deposition time (time) and the current density used. Images are obtained by means of optical microscopy.
Figure 8. Morphology of chitosan coatings/deposits of chitosan obtained on grids with lumen size equal to 280 µm as a function of the deposition time (time) and the current density used. Images are obtained by means of optical microscopy.
Figure 9. Morphology of chitosan coatings/deposits of chitosan obtained on grids with lumen size equal to 140 µm as a function of the deposition time (time) and the current density used. Images are obtained by means of optical microscopy.
Figure 10. Average diameter of the through-pores observed for chitosan coatings/deposits of chitosan on grids with lumen size from 140 to 700 µm as a function of the current density. The data were obtained by averaging 15 measurements. Mean values and standard deviations are reported.
Figure 11. Average diameter of the through-pores observed for chitosan coatings/deposits of chitosan on grids with lumen size equal to 430 µm as a function of the current density and the deposition time (time). The data were obtained by averaging 15 measurements. Mean values and standard deviations are reported.

### Detailed description of the invention

In accordance with the foregoing, a process of manufacturing self-standing films with variable and controlled thickness, characterized by controlled through-porosity micro-patterns, made of natural or synthetic pH-dependent polyelectrolyte macromolecules, is the subject of the present invention; the process comprises:
a) cathodic and/or anodic electrochemical/electrophoretic deposition of said natural or synthetic macromolecules, in particular polysaccharides and/or proteins of natural source, more particularly chitosan and/or alginate and/or collagen, considered individually or in combination with each other, on a working electrode in the form of "conductive micropatterned template", having suitable size/dimensions of the lumen of the meshes, preferably in a conductive material such as graphite or metal, in particular titanium or steel, preferably in the form of grid, the working electrode being immersed in electrolyte solution, preferably aqueous, comprising said macromolecules;
b) mechanical removal of the self-standing films of variable and controlled thickness, characterized by controlled through-porosity micro-pattern obtained in step a) from the working electrode.

By "conductive micropatterned template", as the working electrode according to the present invention, it is meant a conductive substrate having a discontinuous surface, preferably wherein said discontinuity is constituted by holes, preferably through-holes, equally spaced from each other.

Preferably the working electrode according to the present invention is made of conductive material such as graphite or metal, in particular titanium or steel, preferably in the form of a grid. In particular, this working electrode when in the form of a grid is preferably in the form of a grid obtained by weaving and/or machining and/or laser and/or electrochemical machining and/or water-jet. Preferably, the working electrode according to the present invention comprises meshes whose lumen is at least equal to or greater than 1 µm, more preferably at least between 50 and 1000 µm.

More generally it is found that, for the various deposited macromolecules, such as chitosan, an increase in the relative concentration in the electrolyte solution allows the values of the current density applied, the time of deposition and the meshes lumen of the working electrode to be reduced.

The working electrode can be polarized at voltages between 1 mV and 100 V if in potentiostatic conditions or current densities in the range 0.1 - 100 mA/cm², preferably between 1 and 30 mA/cm², can be applied under amperostatic conditions, via a generator of current or DC or AC voltage.

The polarization of the working electrode surface allows a micro-patterned self-standing controlled porosity to be formed, the thickness thereof being modulated by the density of the applied current and the deposition time, therefore the total charge passed.

As an effect of the interface processes to the surface layer, the molecules in solution coagulate in contact with the outer surface of the working electrode, the "conductive micropatterned template", physically depositing on the surface of the working electrode.

The deposition times according to the process subject of the present invention in order to achieve a controlled porosity self-standing micro-pattern comprising of natural or synthetic, pH-dependent, macromolecules, in particular chitosan, are at least equal to or greater than 1 minute, preferably at least comprised between 4 and 40 minutes.

The electrolyte solutions which can be used conveniently for the purposes of the process subject of the present invention are aqueous solutions with a suitable pH and/or solutions with an organic solvent, optionally containing a supporting electrolyte adapted to modify the conductivity of the solution and the electrode overvoltage (acidic in case of chitosan containing anions such as acetate, citrate, maleic acid).

As the preferred natural or synthetic polyelectrolyte macromolecules with pH-dependent solubility which may be conveniently used for the purposes according to the process of the present invention there are natural polysaccharides and proteins, in particular among these chitosan and/or alginate and/or collagen, all considered individually or in combination with each other.

According to the present invention, the concentrations of the macromolecules in the electrolytic solutions are comprised between 0.01 and 10 g/L.

The acid aqueous electrolytic solution with a pH in the range from 2 to 5, containing chitosan in a concentration from 1 to 5 g/L, is particularly preferred.

The process according to the present invention can be carried out in either a discontinuous mode (batch) or a continuous mode.

The process of the invention allows the rate of deposition of macromolecules, the size of the pores, and the degree of interconnection to be modulated in relation to the development of hydrogen, under cathodic conditions, and of oxygen under anodic conditions. The deposition mechanism is also based on the pH-dependence of the solubility of these molecules and the change in pH that is induced by the boundary layer on the surface of properly polarized conductive materials.

By operating according to the electrochemical process of the present invention, self-standing films, characterized by controlled through-porosity micro-patterns made of natural or synthetic, pH-dependant, macromolecules, of variable and controlled thickness, such as chitosan and/or alginate and/or collagen considered individually or in combination with each other, which can be functionalized with drugs, organic and inorganic salts, micro- and nano-particles for including additional functionalities (e.g. pharmacological, antibacterial functionalities, etc.) by co-deposition and/or subsequent deposition.

The process subject of the present invention allows, due to the flexibility and ease of managing and varying parameters of the process: the meshes lumen of the working electrode, pH and nature of the solvents of the electrolyte solution, concentration of the macromolecule(s) to be deposited, the current densities to be employed, with the aim of functionalizing and loading with additives the obtained films by the process according to claim 1 of the present invention, the use of techniques already known in the field of additives and functionalization of 3D porous structures and/or thin film of polyelectrolyte macromolecules such as chitosan and its derivatives. Such techniques can be used either during or after the forming step of the self-standing films with a variable and controlled thickness, characterized by controlled through-porosity micro-patterns made of natural or synthetic, pH-dependent, polyelectrolyte macromolecules.

In particular, through-porosity micro-patterned layers having a dry thickness between 1 µm and 1 mm can be deposited, the through-pores having a circular, rectangular, or other geometric shape, with a size greater than 1 µm.The size of the through pores (through channels) that characterize the micro-pattern of the self-standing film obtained/obtainable by the process according to the present invention is in the range of 50-500 µm, and it is of strong interest for the interaction processes with biological tissues during the healing phase.

The thus deposited films may also be further cross-linked by chemical or physical crosslinking, in order to modulate the kinetics of dissolution or to realize hydrogels with high absorptive power.

Advantageously, according to the process subject of the present invention, in the presence of a cationic polyelectrolyte, such as preferably chitosan, the working electrode acts as a cathode, in the presence of anionic polyelectrolyte, among which preferably alginate, the working electrode acts as an anode.

The films obtained by the process according to claim 1 of the present invention are characterized by pores or through-porosity (through channels) oriented according to the direction perpendicular to the film plane and with ordered and regular geometry in the same plane (Micro-pattern), said pores or through-porosity being controlled and controllable in shape, distribution in micrometer-size of channels and spacing thereof. The material constituting the films obtained by the process according to claim 1 of the present invention being further characterized by a random porosity, non-through pores (secondary porosity), by a size smaller than the channels or through-pores, and less than 50% of the film thickness, said secondary porosity being a consequence of the deposition process of the film according to the present invention.

It is a further object of the present invention a self-standing film, with a variable and controlled thickness, characterized by controlled through-porosity micro-patterns, made of natural or synthetic, pH-dependent, polyelectrolyte macromolecules, preferably said macromolecules being selected among natural polysaccharides and/or proteins; more preferably they are selected among chitosan and/or alginate and/or collagen, considered individually or in combination between them, wherein said pores or through-porosity (through channels) are oriented according to the perpendicular direction to the film plane and with an ordered and regular geometry in the same plane (Micro-pattern), said through-pores or porosity (primary porosity) being controlled and controllable in shape, in distribution, and in the micrometer-size of channels and spacing thereof, with the material of the film being characterized by a random porosity, non-through pores (porosity secondary) of a smaller size than channels or through-pores, in which the ratio between the number of through-pores (primary porosity) and non-through pores (secondary porosity) results preferably in a relative density comprised between 0.3 and 1, with respect to the material constituting the film, in particular when said material comprises or consists of chitosan.

Advantageously, according to the process subject of the present invention between step a) of deposition and the step b) of removal it is optionally provided a neutralization step for subsequent washes of the working electrode, on which the self-standing film with variable and controlled thickness characterized by a controlled through-porosity micro-pattern, is deposited, typically by immersion in neutral pH baths, possibly preceded by an alkaline pH wash, if the deposition took place in an acid environment or vice-versa, then followed by removal of the washing solvent.

According to the process subject of the present invention, in case the macromolecule polyelectrolyte is chitosan, the latter is insoluble in water and in organic solvents; however, the protonated chitosan can be dissolved in water at lower pH. The protonation of the amine groups of chitosan may take place in acidic solutions:

Chit-NH₂ + H₃O⁺ ⇔ Chit-NH₃⁺ + H₂O (Eq.1)

At low pH, chitosan becomes a cationic polyelectrolyte. Its pH-dependent solubility in aqueous solutions allows to work whereas a slight increase in pH towards neutrality allows to obtain different forms (for example, membranes or films) by processes such as electrophoretic deposition: under the action of an electric field, the chitosan charged macromolecules move toward the cathode.

The cathodic reactions below occur at alkaline pH on the electrode surface:

2H₂O + 2e⁻ ⇔ H₂ + 2OH⁻

O₂ + 2H₂O + 4e⁻ ⇔ 4OH⁻

NO₃⁻ + 7H₂O + 8e⁻ ⇔ NH₄⁺ +10 OH⁻

In these conditions the protonated chitosan deprotonates in the proximity of the cathode:

Chit-NH₃⁺ + OH⁻ ⇔ Chit-NH₂ + H₂O

The change of pH is caused by the electrolysis of water: the cathodic reactions in aqueous solutions cause a net production of OH⁻ ions, and then a pH gradient. If the electrolysis is carried out in the presence of chitosan, the polymer chains distant from the electrode experience a low pH (less than 6) and therefore they are protonated and soluble, while the chains that are closer to the cathode experience a higher pH and then are less protonated and less soluble; the chains in proximity of the electrode are deprotonated and then become insoluble, since the pH is greater than 7, and these chains are deposited in the form of a hydrogel film. This is stable in the absence of an applied potential and at pH greater than 6.5.

The thickness, the spatial resolution, and the morphology of electrodeposited chitosan can be controlled by the deposition conditions such as the concentration of chitosan in the electrolyte solution, the voltage applied to the electrodes, and the time. Thin films or hydrogels of chitosan can be obtained and the porosity of the deposit can be controlled, by varying the current density.

The knowledge of the electrophoretic mobility of the molecules of chitosan is necessary to control the deposition rate and to have flexibility in manipulating microstructural coatings of chitosan. The electro-migration of D-glucosamine and oligomers of chitosan is pH-dependent, which influences the weight of the deposit area and the deposition rate. Also the conductivity of the electrolytic solution is pH dependent because the degree of dissociation of the chitosan, which is soluble or insoluble according to the charges exhibited by the amine groups that are protonated or deprotonated if the pH is lower or higher than their pKₐ, depends thereon. Not only the thickness or the weight of the deposit are affected by the pH, but also its porosity. In fact by increasing the deposition rate, the reaction of hydrogen evolution at the cathode proceeds faster, causing pores in the deposit of chitosan to be formed.

The electrodeposition of chitosan constitutes a simple and fast method, which requires simple apparatus and short processing times to assemble components for biosensors and for the transduction of signals, for the biofunctionalization of surfaces and to create vesicles and composite coatings for tissue engineering even complex surfaces.

In order to better understand the present invention and to practice the same, some illustrative examples are shown below; however, they should not be considered in any way as limiting the scope of the invention itself.

### EXAMPLES

### Comparative example.

### Homogeneous film of chitosan deposited on the cathode in titanium continuous substrate.

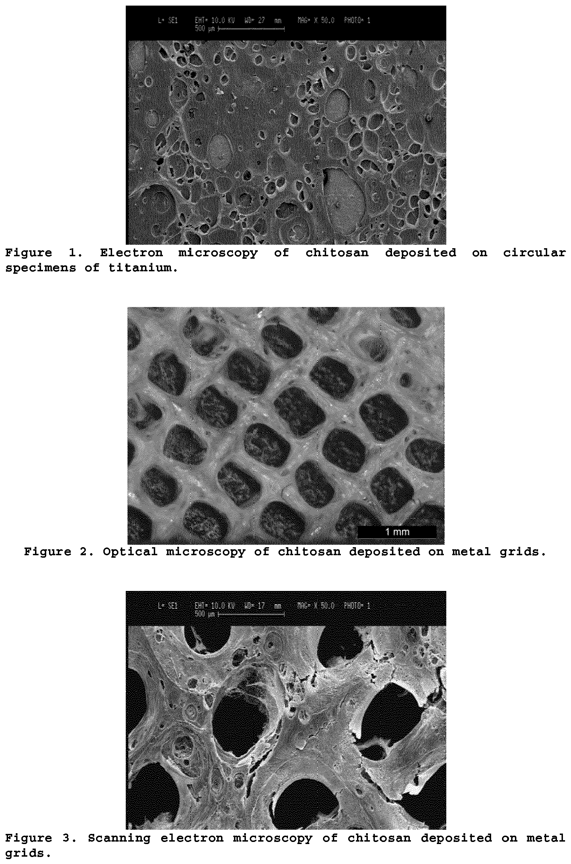

The electrochemical deposition of chitosan on the cathode was performed using a continuous titanium substrate, with deposition time of 4 minutes, current density of 20 mA/cm², concentration of chitosan equal to 1g/L, the pH of the aqueous electrolyte solution containing chitosan being equal to 3.5. The deposit obtained under the above conditions on continuous titanium roller is illustrated in Figure 1, wherein it may be seen that under controlled conditions a porous deposit can be obtained. The arrangement of the pores on the substrate, though uniform, is random and unordered.

All the images here reported by both scanning electron microscopy and optical microscopy, were obtained respectively by scanning electron microscope Cambridge Stereoscan 360, 200x-5000x magnification, optical microscope LEICA DM LM Magnification 50x-500x magnification and stereo microscope WILD M8 5x-50x.

### Examples according to the invention.

### Self-standing controlled porosity micro-pattern deposited on cathode in the form of steel metal grids with different lumens of the mesh.

This series of samples of self-standing controlled porosity micro-pattern were prepared on cathodes in the form of steel grids with different mesh lumens; lumens respectively of 140 µm, 280 µm, 430 µm, 560 µm and 700 µm. For each type of grid both the applied current density and the relative time of application were varied, whereas in all the tests the conditions of concentration of chitosan remained identical: 1g/L and pH 3.5 of the aqueous electrolyte solution containing chitosan and in which the working electrodes were immersed.

An example of the self-standing controlled porosity micro-pattern obtained using metallic grids is illustrated in Figure 2 (optical microscopy) and Figure 3 (scanning electron microscopy). In Figures 2 and 3 it is observed that the deposit of chitosan is porous. The through-pores are arranged in a regular pattern and they replicate the underlying metal grid. From electron microscopy images the presence of a secondary porosity characterized by pores of lower size interposed between the pores of larger size/dimensions with a regular arrangement is observed. As to the deposition on the metal grids, the parameters concerning the electrolytic solution (pH and concentration of chitosan) were kept unchanged while the parameters of the current density and deposition time were changed. The effect of these two parameters on the deposited mass and on the morphology and porosity of the obtained deposits has been evaluated by taking into account the variation of the lumen size in the substrate.

The deposits obtained on the grids were observed under the optical microscope to check the morphology of the same in order to understand how the deposition parameters affected it.

The results can be summarized simply in matrices of images. The abscissa shows the time taken for the deposition (in minutes) while the ordinate indicates the variation of the current density (mA/cm²).

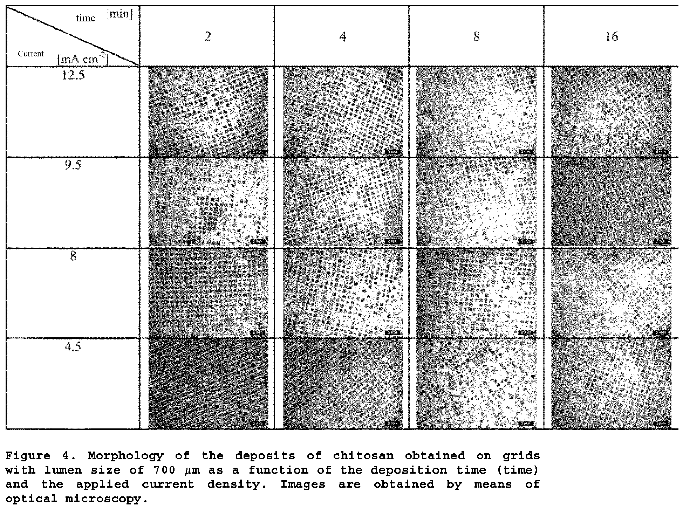

Figure 4 shows a good distribution of the deposit on the cathode surface: upon increase of the deposition time the amount of chitosan deposited increases. Furthermore, such behaviour can be also highlighted upon the increase of current density.

Figures 4 to 9 show matrices of images obtained by means of stereomicroscopy to observe in further details the morphology of the deposits on the grids upon varying the size of the lumen.

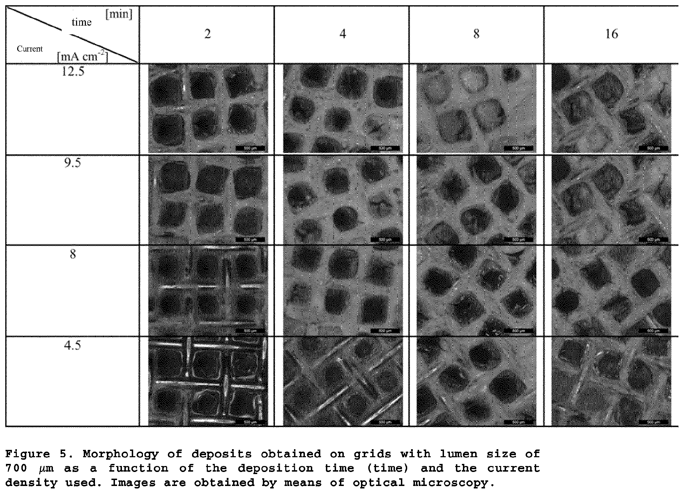

In Figure 5 it is observed that deposited chitosan increases as a function of the deposition time and current density. The pores are present on all the substrates except for depositions of two minutes, 4.5 and 8 mA/cm².

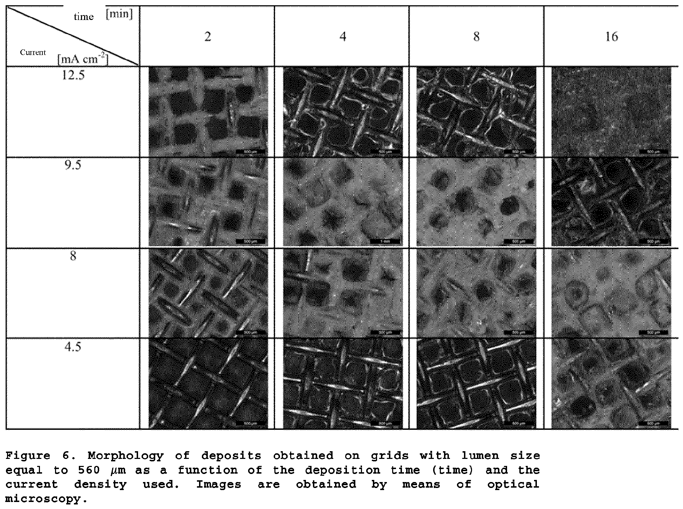

In Figure 6 the matrix of images is shown, which describes the deposition of metallic grids obtained with the lumen size equal to 560 µm.In Figure 6 it is observed that the deposited chitosan increases when the current density increases up to 9.5 mA/cm². At 12.5 mA/cm² the amount of deposited chitosan appears to decrease. On all the substrates the presence of through-pores and closed pores is noted.

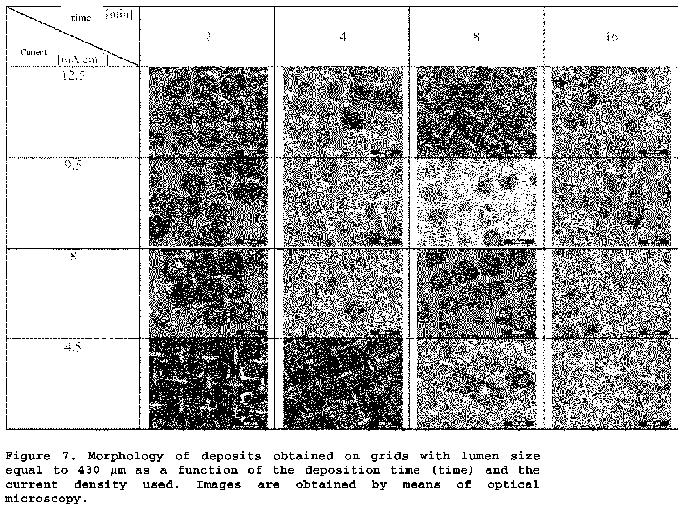

In Figure 7 the matrix of images is shown, which describes the deposits obtained on metal grids with lumen size equal to 430 µm. In Figure 7 it is observed that the presence of through-pores only for current density equal to 8 and 9.5 mA/cm² and deposition times of less than 16 minutes may be detected. In other cases the deposit of chitosan appears as a continuous film.

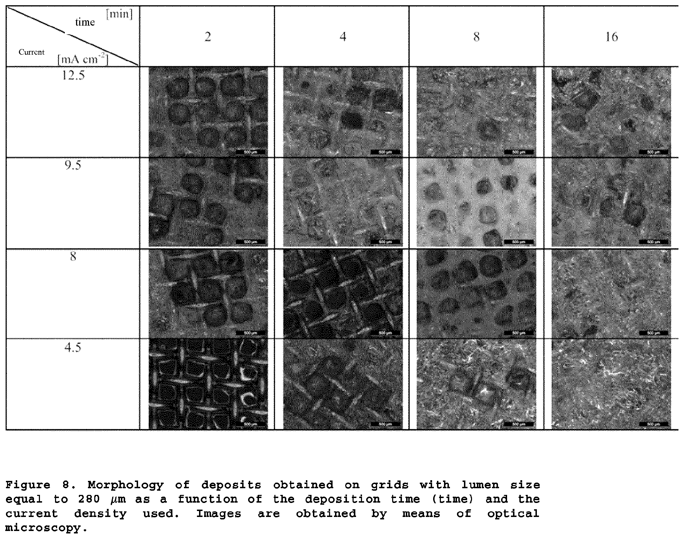

Figure 8 shows the matrix of images that describes the deposits of chitosan obtained on metal grids with lumen size equal to 280 µm. In Figure 8 it is observed that the presence of through-pores only for depositions of 8 minutes and current density equal to 8 or 9.5 mA/cm² may be detected. For longer deposition times a continuous film of chitosan is seen. For shorter deposition times the deposit, if present, has no through pores.

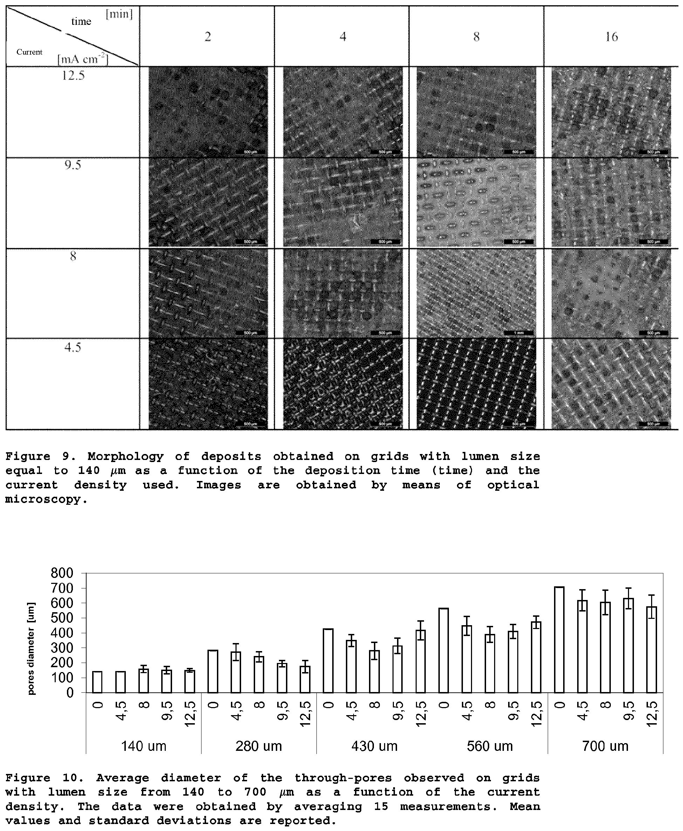

Figure 9 shows the matrix of images of deposits of chitosan on metal grids with the lumen size equal to 140 microns. In Figure 9 it is observed that for deposition times equal to 2 minutes and current density of 4.5 mA/cm² there is no deposit of chitosan. In other cases the coating appears as a continuous film and not as a surface provided with through-pores.

From Figures 4, 5, 6, 7, 8 and 9 it may be observed that:
1. By decreasing the lumen size, the size of the pores also decreases and in particular, while these are easily observable for the substrates with lumen up to 560 µm, lower size/dimensions require a more careful optimization of the process
2. In all cases, it is observed that for longer deposition times, films are continuous, while for shorter deposition times deposited chitosan seems only to cover the metal grid and does not form any through-pores.
3. In all cases, a hierarchy of porosity may be observed: the primary one provided by through-pores and a secondary porosity with a pore size which appears to be lower in size/dimensions and less uniform than that of the through-pores, probably attributable to the development at the cathode of hydrogen during the deposition of chitosan.
4. For low current densities or low deposition times, chitosan appears to cover the grid and the pores are not circular in shape.
5. Upon the increase of the amount of the deposited chitosan, pores first tend to assume a cylindrical shape and then they keep a constant diameter.

For the statistical analysis of the pore size, the samples subjected to electrophoretic treatment were observed by a fluorescence optical microscope. Five images were acquired for each sample and for each image three measurements were performed. The graphs correlate the average size of the pores of each deposit with the intensity of current upon the variation of the size of the substrate lumen.

From the graph in Figure 10, it is possible to notice that the average size of the pores is influenced by the size of the lumen of substrate. Furthermore, depending on the size of the grid, there seems to be a different behaviour at different current densities. In particular for smaller meshes, pore size decreases as the apparent density of current increases. For meshes of 430 and 560 µm, after an initial decrease of the pore size, it increases, while for 700 µm it seems no influence by the current density exists. In any case a reduction in size of channels in respect to the size of the grid lumen (value 0) may be noted. The measurement on grids with lumen size equal to 140 µm with a current intensity of 4.5 mA/cm² could not be taken, because the chitosan had not deposited on the substrate. The initial value of the grid lumen is then shown in the graph.

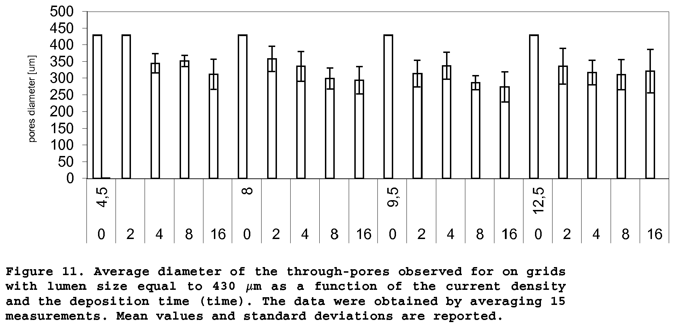

In order to investigate the pore size upon the variation of the deposition time, an analysis was performed on grids with a lumen size equal to 430 µm as an example. The results are reported in the graph in Figure 11. In Figure 11, it is observed that, for each current density, the pore size decreases as the time increases. The pore size does not seem to be influenced by the current density while it decreases when the deposition time increases.

## Claims

1. Process of manufacturing of self-standing films with variable and controlled thickness, **characterized by** controlled though-porosity micro-patterns made of natural or synthetic, pH-dependent, polyelectrolyte macro-molecules, the process comprising:
a) cathodic and/or anodic electrochemical/electrophoretic deposition of said natural or synthetic macromolecules, on a working electrode in the form of conductive micropatterned template, conductive substrate having a discontinuous surface, wherein said discontinuity is constituted by through-holes, equally spaced from each other, having suitable size/dimensions of the lumen of the meshes, the working electrode immersed in an electrolyte solution comprising said macromolecules, the polarization of the working electrode surface allows a micro-patterned self-standing controlled porosity to be formed, as an effect of the interface processes to the surface layer, the molecules in solution coagulate in contact with the outer surface of the working electrode, the "conductive micropatterned template", physically depositing on the surface of the working electrode;
b) mechanical removal of the self-standing films of variable and controlled thickness **characterized by** a controlled through-porosity micro-pattern obtained in step a) from the working electrode.

2. Process according to claim 1, wherein the polyelectrolyte macromolecules are selected from natural polysaccharides and/or proteins.

3. Process according to claim 1, wherein the polyelectrolyte macromolecules are selected from chitosan and/or alginate and/or collagen, considered individually or combinations thereof.

4. Process according to claim 1, wherein the working electrode is made of a conductive material such as graphite or metal, in particular titanium or steel.

5. Process according to claim 1, wherein the working electrode is in the form of a grid.

6. Process according to claim 1, wherein the electrolyte solution is an aqueous solution.

7. Process according to claim 1, wherein the current density is comprised in the range of 0.1 - 100 mA/cm².

8. Process according to claim 1, wherein the concentration of macromolecules is comprised between 0.01 and 10 g/L.

9. Self-standing film obtained by the process according to claim 1, said film having a variable and controlled thickness, **characterized by** controlled though-porosity micro-pattern made of natural or synthetic, pH-dependent, polyelectrolyte macro-molecules, wherein said pores or through-porosity (through-channels) are oriented according to the perpendicular direction to the film plane and with an ordered and regular geometry in the same plane (Micro-pattern), said through-pores or porosity (primary porosity) being controlled and controllable in shape, distribution and micrometric size/dimensions of the channels and spacing thereof, the material constituting the film being **characterized by** a random porosity, non through-pores (secondary porosity), of smaller size/dimensions than channels or through-pores, wherein the ratio between the number of through-pores (primary porosity) and non through-pores (secondary porosity) preferably results in a relative density comprised between 0.3 and 1 with respect to the material forming the film.

10. Film according to claim 9, wherein the polyelectrolyte macromolecules are selected from natural polysaccharides and/or proteins.

11. Film according to claim 9, wherein the polyelectrolyte macromolecules are selected from chitosan and/or alginate and/or collagen considered individually or combinations thereof.

## Patentansprüche

1. Verfahren zur Herstellung von freistehenden/selbststehende Filmen mit variabler und kontrollierter Dicke, **gekennzeichnet durch** Mikromuster mit Durchgangsporosität aus natürlichen oder synthetischen, pH-abhängigen Polyelektrolyt-Makromolekülen, wobei das Verfahren umfasst:
a) kathodische und/oder anodische elektrochemische/elektrophoretische Abscheidung der natürlichen oder synthetischen Makromoleküle auf einer Arbeitselektrode in Form einer leitenden mikrostrukturierten Schablone, wobei ein leitendes Substrat eine diskontinuierliche Oberfläche hat, wobei die Diskontinuität aus gleichmäßig voneinander beabstandeten Durchgangslöchern mit einer geeigneten Größe/geeigneten Abmessungen des Lumens der Maschen gebildet wird, wobei die Arbeitselektrode in eine Elektrolytlösung eingetaucht ist, die die Makromoleküle umfasst, wobei die Polarisation der Arbeitselektrodenoberfläche die Bildung einer mikrostrukturierten selbstständigen kontrollierten Porosität als Wirkung der Grenzflächenprozesse auf die Oberflächenschicht ermöglicht, die Moleküle in Lösung in Kontakt mit der Außenfläche der Arbeitselektrode, der "leitenden mikrostrukturierten Schablone", koagulieren, und sich physisch auf der Oberfläche der Arbeitselektrode ablagern;
b) mechanische Entfernung der freistehenden/selbststehenden Filme mit variabler und kontrollierter Dicke, **gekennzeichnet durch** ein in Schritt a) von der Arbeitselektrode erhaltenes Mikromuster mit kontrollierter Durchgangsporosität.

2. Verfahren nach Anspruch 1, wobei das Polyelektrolyt-Makromolekül aus natürlichen Polysacchariden und/oder Proteinen ausgewählt ist.

3. Verfahren nach Anspruch 1, wobei das Polyelektrolyt-Makromolekül einzeln aus Chitosan und/oder Alginat und/oder Kollagen oder Kombinationen davon ausgewählt ist.

4. Verfahren nach Anspruch 1, wobei die Arbeitselektrode aus einem leitenden Material wie Graphit oder Metall, insbesondere aus Titan oder Stahl, hergestellt ist.

5. Verfahren nach Anspruch 1, wobei die Arbeitselektrode in der Form eines Gitters vorliegt.

6. Verfahren nach Anspruch 1, wobei die Elektrolytlösung eine wässrige Lösung ist.

7. Verfahren nach Anspruch 1, wobei die Stromdichte im Bereich von 0,1 - 100 mA/cm² liegt.

8. Verfahren nach Anspruch 1, wobei die Konzentration von Makromolekülen zwischen 0,01 und 10 g/l liegt.

9. Freistehender/selbststehender Film, erhalten durch das Verfahren nach Anspruch 1, wobei der Film eine variable und kontrollierte Dicke aufweist, die durch ein Mikromuster mit kontrollierter Durchgangsporosität gekennzeichnet ist, das aus natürlichen oder synthetischen, pH-abhängigen Polyelektrolyt-Makromolekülen hergestellt ist, wobei die Poren oder die Durchgangsporosität (Durchgangskanäle) entsprechend der senkrechten Richtung zur Filmebene und mit einer geordneten und regelmäßigen Geometrie in derselben Ebene (Mikromuster) ausgerichtet sind, wobei die Durchgangsporen oder die Porosität (Primärporosität) gesteuert werden und in Form, Verteilung und mikrometrischer Größe/Abmessungen der Kanäle und deren Abstand steuerbar sind, wobei das Material, aus dem der Film besteht, durch eine zufällige Porosität und nicht-durchgehende Poren (sekundäre Porosität) von geringerer Größe/Abmessungen als Kanäle oder Durchgangsporen gekennzeichnet ist, wobei das Verhältnis zwischen der Anzahl der Durchgangsporen (primäre Porosität) und der nichtdurchgehenden Poren (sekundäre Porosität) vorzugsweise zu einer relativen Dichte zwischen 0,3 und 1 in Bezug auf das Material, das den Film bildet, führt.

10. Film nach Anspruch 9, wobei die Polyelektrolyt-Makromoleküle aus natürlichen Polysacchariden und/oder Proteinen ausgewählt sind.

11. Film nach Anspruch 9, wobei die Polyelektrolyt-Makromoleküle einzeln aus Chitosan und/oder Alginat und/oder Kollagen oder Kombinationen davon ausgewählt sind.

## Revendications

1. Procédé de fabrication de films autoportants d'épaisseur variable et régulée, **caractérisé par** des micro-motifs à porosité traversante régulée constitués de macromolécules de polyélectrolytes naturels ou synthétiques, dépendant du pH, le procédé comprenant :
a) le dépôt électrochimique/électrophorétique cathodique et/ou anodique desdites macromolécules naturelles ou synthétiques, sur une électrode de travail sous la forme d'un gabarit conducteur à micro-motifs, d'un substrat conducteur ayant une surface discontinue, ladite discontinuité étant constituée par des trous traversants, également espacés les uns des autres, ayant une taille/des dimensions appropriées de la lumière des mailles, l'électrode de travail étant immergée dans une solution électrolytique comprenant lesdites macromolécules, la polarisation de la surface de l'électrode de travail permettant de former une porosité régulée autonome à micro-motifs, comme un effet des processus d'interface avec la couche superficielle, les molécules en solution coagulant au contact de la surface extérieure de l'électrode de travail, le « gabarit conducteur à micro-motifs », se déposant physiquement sur la surface de l'électrode de travail ;
b) l'élimination mécanique des films autoportants d'épaisseur variable et régulée **caractérisée par** un micro-motif à porosité traversante régulée obtenu à l'étape a) depuis l'électrode de travail.

2. Procédé selon la revendication 1, dans lequel les macromolécules polyélectrolytes sont choisies parmi les polysaccharides naturels et/ou les protéines.

3. Procédé selon la revendication 1, dans lequel les macromolécules polyélectrolytes sont choisies parmi le chitosane et/ou l'alginate et/ou le collagène, considérées individuellement ou leurs combinaisons.

4. Procédé selon la revendication 1, dans lequel l'électrode de travail est réalisée en un matériau conducteur tel que le graphite ou le métal, notamment le titane ou l'acier.

5. Procédé selon la revendication 1, dans lequel l'électrode de travail se présente sous la forme d'une grille.

6. Procédé selon la revendication 1, dans lequel la solution d'électrolyte est une solution aqueuse.

7. Procédé selon la revendication 1, dans lequel la densité de courant est comprise dans la plage de 0,1 à 100 mA/cm².

8. Procédé selon la revendication 1, dans lequel la concentration de macromolécules est comprise entre 0,01 et 10 g/l.

9. Film autoporteur obtenu par le procédé selon la revendication 1, ledit film ayant une épaisseur variable et régulée **caractérisée par** un micro-motif à porosité traversante régulée faite de macromolécules de polyélectrolyte naturel ou synthétique, dépendant du pH, dans lequel lesdits pores ou ladite porosité traversante (canaux traversants) sont orientés selon la direction perpendiculaire au plan du film et avec une géométrie ordonnée et régulière dans le même plan (micro-motif), lesdits pores traversants ou porosité (porosité primaire) étant régulés en forme, distribution et taille/dimensions micrométriques des canaux et de leur espacement, le matériau constituant le film étant **caractérisé par** une porosité aléatoire, des pores non traversants (porosité secondaire), de taille/dimensions plus petites que les canaux ou pores traversants, le rapport entre le nombre de pores traversants (porosité primaire) et de pores non traversants (porosité secondaire) résultant de préférence en une densité relative comprise entre 0,3 et 1 par rapport au matériau formant le film.

10. Film selon la revendication 9, dans lequel les macromolécules polyélectrolytes sont choisies parmi les polysaccharides naturels et/ou les protéines.

11. Film selon la revendication 9, dans lequel les macromolécules polyélectrolytes sont choisies parmi le chitosane et/ou l'alginate et/ou le collagène considérés individuellement ou leurs combinaisons.
